# EUROPEAN PATENT SPECIFICATION

(11) **EP 1 573 046 B1**
(45) Date of publication and mention of the grant of the patent: **09.02.2011**
(21) Application number: 03793134.2
(22) Date of filing: 19.08.2003
(51) Int. Cl.: A01N 43/04, C07K 14/60, A61K 38/25

(54) **PROTEASE RESISTANT TI-GROWTH HORMONE RELEASING HORMONE**
PROTEASERESISTENTES TI-WACHSTUMSHORMON FREISETZENDES HORMON
HORMONE DE LIBERATION DE L'HORMONE DE CROISSANCE TI RESISTANT AUX PROTEASES

(30) Priority: 21.08.2002 US 166356
(43) Date of publication of application: 14.09.2005
(73) Proprietor: BAYLOR COLLEGE OF MEDICINE, Houston, TX 77030 (US)
(72) Inventor: DRAGHIA-AKLI, Ruxandra, Houston, TX 77035 (US); FIOROTTO, Marta, L., Houston TX 77005 (US); TAFFET, George, Houston, TX 77025-1715 (US)
(74) Representative: Bassil, Nicholas Charles
(86) International application number: PCT/US2003/025975
(87) International publication number: WO 2004/018697

(56) References cited:
- WO-A-01/06988
- WO-A-87/06835
- WO-A2-03/038112
- DRAGHIA-AKLI RUXANDRA ET AL: "Myogenic expression of an injectable protease-resistant growth hormone-releasing hormone augments long-term growth in pigs" NATURE BIOTECHNOLOGY, NATURE PUBLISHING GROUP, NEW YORK, NY, US, vol. 17, no. 12, December 1999 (1999-12), pages 1179-1183, XP002159711 ISSN: 1087-0156
- FROMAN LA ET AL: 'Dipeptidylpeptidase IV and Trypsin-like Enzymatic Degradation of Human Growth Hormone-releasing Hormone in Plasma.' J INVEST. vol. 83, no. 5, May 1989, pages 1533 - 1540, XP002993666
- MARTIN RA ET AL: 'Dipeptidyl peptidase IV (DPP-IV) from pig kidney cleaves analogs of bovine growth hormone-releasing factor (bGRF) modified at position 2 with Ser, Thr or Val. Extended DPP-IV substrate specificity?' BIOCHIMICA ET BIOPHYSICA ACTA. vol. 1164, no. 3, 1993, pages 252 - 260, XP000853614
- COLAO A ET AL: "Improved Cardiovascular Risk Factors and Cardiac Performance after 12 Months of Growth Hormone (GH) Replacement in Young Adult Patients with GH Deficiency" THE JOURNAL OF CLINICAL ENDOCRINOLOGY & METABOLISM, vol. 86, no. 5, 1 May 2001 (2001-05-01), pages 1874-1881,
- VALCAVI R ET AL: "Cardiac performance and mass in adults with hypopituitarism: effects of one year of growth hormone treatment" THE JOURNAL OF CLINICAL ENDOCRINOLOGY & METABOLISM, vol. 80, pages 659-666,
- GOLDSTEIN M ET AL: "Evaluation of cardiac function by echo-Doppler studies in critically ill patients" INTENSIVE CARE MED, vol. 14, 1 January 1988 (1988-01-01), pages 406-410,

## Description

### BACKGROUND

One aspect of the current invention is a composition comprising a modified growth hormone releasing hormones ("GHRH") molecule for use as a medicament in improving cardiac function, as assessed by Doppler echocardiogram. The composition may also be a nucleic acid molecule that encodes the modified growth hormone releasing hormone ("GHRH"). The modified GHRH can be defined as a biologically active polypeptide that has been engineered to contain a distinct amino acid sequence while simultaneously having similar or improved biologically activity when compared to a wild-type GHRH ("wt-GHRH") polypeptide. One benefit of the claimed invention occurs when the modified molecule with the GHRH composition is delivered to a subject. The modified GHRH increases the level of growth hormone (GH") secretion in a subject. Other benefits outlined in a preferred embodiment include improved cardiac function in aging mammals. The modified GHRH composition of this invention is also resistant to degradation compared to the wt-GHRH.

Regulated expression of the growth hormone ("GH") pathway is essential for optimal linear growth, as well as homeostasis of carbohydrate, protein, and fat metabolism. GH synthesis and its pulsatile secretion from the anterior pituitary is stimulated by growth hormone releasing hormone ("GHRH") and inhibited by somatostatin, both hypothalamic hormones (Frohman et al., 1992). GH increases production of insulin-like growth factor-I (IGF-I) primarily in the liver, as well as other target organs. IGF-I and GH feedback on the hypothalamus and pituitary to inhibit GHRH release and GH secretion. The endogenous rhythm of GH secretion becomes entrained to the imposed rhythm of exogenous GHRH (Caroni and Schneider, 1994).

Linear growth velocity and body composition respond to GH or GHRH replacement therapies in a broad spectrum of conditions, both in humans and in farm animals. The etiology of these conditions can vary significantly. In 50% of human GH deficiencies the GHRH-GH-IGF-I axis is functionally intact, but does not elicit the appropriate biological responses in its target tissues. Similar phenotypes are produced by genetic defects at different points along the GH axis (Parks et al., 1995), as well as in non-GH-deficient short stature. In the non-GH-deficiency causes of short stature, such as Turner syndrome (Butler et al., 1994), hypochondroplasia (Foncea et al., 1997), Crohn's disease (Parrizas and LeRoith, 1997), intrauterine growth retardation (Hoess and Abremski, 1985) or chronic renal insufficiency (Lowe, Jr. et al., 1989), GHRH or GH therapy can be effective in promoting linear growth (Gesundheit and Alexander, 1995). In the elderly, the GHRH-GH-IGF-I axis undergoes considerable decrement, with reduced GH secretion and IGF-I production associated with a loss of skeletal muscle mass (sarcopenia), osteoporosis, increased fat deposition and decreased lean body mass (Caroni and Schneider, 1994; Veldhuis et al., 1997). It has been demonstrated that the development of these changes can be offset by recombinant GH therapy. GH replacement therapy both in children and the elderly is widely used clinically. Current GH therapy has several shortcomings, however, including frequent subcutaneous or intravenous injections, insulin resistance and impaired glucose tolerance (Rabinovsky et al., 1992); children are also vulnerable to premature epiphyseal closure and slippage of the capital femoral epiphysis (Liu and LeRoith, 1999). A "slow-release" form of GH (Genentech), which requires injections every 14 days, perturbs the normal physiological pulsatile GH profile, and is also associated with frequent side effects.

In domestic livestock, GHRH and GH stimulate milk production, with an increase in feed to milk conversion, which additionally enhances growth, primarily by increasing lean body mass (Lapierre et al., 1991; van Rooij et al., 2000) with overall improvement in feed efficiency. Hot and chilled carcass weights are increased and carcass lipid (percent of soft-tissue mass) is decrease by GHRH and GH (Etherton et al, 1986).

Administering GHRH analog proteins (U.S. Pat Nos. 5,847,066; 5846,936; 5,792,747; 5,776,901; 5,696,089; 5,486,505; 5,137.872; 5,084,442, 5,036,045; 5,023,322; 4,839,344; 4,410,512, RE33.699), synthetic or naturally occurring peptide fragments of GHRH (U.S. Pat. Nos, 4,833,166; 4,229,158; 4,228,156; 4,226,857; 4,224,316; 4,223,021; 4,223,020; 4,223, 019) for the purpose of increasing release of growth hormone have been reported. GHRH analogs containing the following mutations have been reported (U.S. Patent No. 5,846,936): Tyr at position 1 to His; Ala at position 2 to Val, Leu, or others; Asn at position 8 to Gln, Ser, or Thr; Gly at position 15 to Ala or Leu; Met at position 27 to Nle or Leu; and Ser at position 28 to Asn. The GHRH analog is the subject of U.S. Patent Application Serial No. 09/624,268 ("the '268 patent"), which teaches application of a GHRH analog containing mutations that improve the ability to elicit the release of growth hormone. In addition, the '268 patent relates to the treatment of growth deficiencies; the improvement of growth performance; the stimulation of production of growth hormone in an animal at a greater level than that associated with normal growth; and the enhancement of growth utilizing the administration of growth hormone releasing hormone analog, Valcavi *et al,* 1995, studied the effect of growth hormone (GH) administration on myocardial structure and function in 20 GH-deficient patients with hypopituitarism.

Although GHRH protein therapy stimulates normal cyclical GH secretion with virtually no side effects (Corpas et al., 1993), the short half-life of the molecule *in vivo* requires frequent (one to three times per day) intravenous, subcutaneous or intranasal (at 300-fold higher dose) administrations. Thus, recombinant GHRH administration is not practical as a chronic therapy. However, extracranially secreted GHRH, as a mature or a truncated polypeptide, is often biologically active (Thorner et al., 1984) and a low level of serum GHRH (100pg/ml) stimulates GH secretion (Corpas et al. 1993). These characteristics make GHRH an excellent candidate for plasmids mediated supplementation of a gene product.

Gene Delivery and *in vivo* Expression: Recently, the delivery of specific genes to somatic tissue in a manner that can correct inborn or acquired deficiencies and imbalances was proved to be possible. Gene-based drug delivery offers a number of advantages over the administration of recombinant proteins. These advantages include the conservation of native protein structure, improved biological activity, avoidance of systemic toxicities, and avoidance of infectious and toxic impurities. In addition, gene therapy allows for prolonged exposure to the protein in the therapeutic range, because the protein is synthesized and secreted continuously into the circulation.

The primary limitation of using recombinant protein is the limited availability of protein after each administration. Gene therapy using injectable DNA plasmid vectors overcomes this, because a single injection into the patient's skeletal muscle permits physiologic expression of the protein for extensive periods of time (WO 99/05300 and WO 01/06988). Injection of the vectors promotes the production of enzymes and hormones in animals in a manner that more closely mimics the natural process. Furthermore, among the non-viral techniques for gene transfer *in vivo,* the direct injection of plasmid DNA into muscle tissue is simple, inexpensive, and safe.

Direct plasmid DNA transfer is currently the basis of many emerging therapeutic strategies, as it avoids the potential problems associated with viral genes or lipid particles (Muramatsu et al., 1998). Skeletal muscle is a preferred target tissue, because the muscle fiber has a long life span and can be transduced by circular DNA plasmids. Skeletal muscle borne plasmids have been expressed efficiently over months or years in immunocompetent hosts (Davis et al., 1993; Tripathy et al., 1996). Plasmid DNA constructs are attractive candidate for direct therapy into the subjects skeletal muscle because they are well-defined entities, which are biochemically stable and have been used successfully for many years (Acsadi et al., 1991; Wolff et al., 1990). The relatively low expression levels, achieved after direct plasmid DNA injection are sometimes sufficient to prove bio-activity of secreted peptides (Danko and Wolff, 1994; Tsurumi et al., 1996).

Previously, we reported that in mice, human GHRH cDNA could be delivered to muscle by an injectable myogenic expression vector, where it transiently stimulated GH secretion over a period of two weeks (Draghia-Akli et al., 1997). We have then optimized this injectable vector system by incorporating a powerful synthetic muscle promoter (Li et al., 1999) coupled with a novel protease-resistant GHRH molecule with a substantially longer half life and greater GH secretory activity (pSP-HV-GHRH) (Draghia-Akli et al., 1999). We improved vector delivery to skeletal muscle via a highly efficient electroporation technology (Wang et al., 1998). Using this combination of vector design and electric pulses plasmid delivery method, we were able to show increased growth and favorably modified body composition in pigs (Draghia-Akli et al., 1999) and rodents (Draghia-Akli et al., 2002). The current invention describes a new protease resistant, super-active GHRH analog, called TI-GHRH.

### SUMMARY

One aspect of the current invention is a composition comprising a modified growth hormone releasing hormone (GHRH") for use as a medicament in improving cardiac function, as assessed by Doppler echocardiogram.
A preferred embodiments of this invention includes a peptide with a general formula (-A₁-A₂-DAIFTNSYRKVL-A₃-QLSARKLLQDI-A₄-A₅-RQQGERNQEQGA-OH, wherein A₁ is a D-or L-isomer of the amino acid tyrosine ("Y"), or histidine ("H"); A₂ is a D-or L-isomer of the amino acid alanine ("A"), valine ("V"), or isoleucine ("T"); A₃ is a D-or L-isomer of the amino acid alanine ("A") or glycine ("G"); A₄ is a D-or L-isomer of the amino acid methionine ("M"), or leucine ("L"); A₅ is a D-or L-isomer of the amino acid serine ("S") or asparagine ("N"). Other preferred embodiments include a modified GHRH as shown in Seq ID #3, Seq ID #4, and Seq ID #5. The modified GHRH can be defined as a biologically active polypeptide that was engineered to contain a distinct amino acid sequence while simultaneously having similar or improved biologically activity compared to the wild-type GHRH ("wt-GHRH") polypeptide. One benefit of the claimed invention occurs when the modified GHRH, composition is delivered to a subject. The modified GHRH increases the level of growth hormone ("GH") secretion in a subject. The preferred subject is a domesticated animal or human. Other benefits outlined in a preferred embodiment include improved cardiac function in aging mammals. Additionally, the modified GHRH composition is resistant to degradation when compared to the wt-GHRH.

Another aspect of the current invention is a nucleic acid molecule (e.g. DNA or RNA) that encodes the modified growth hormone releasing hormone ("GHRH") for use as medicament in improving cardiac function, as assessed by Doppler echocardiogram. In preferred embodiment, nucleic acid molecule further comprises a synthetic mammalian expression plasmid with a synthetic or eukaryotic promoter; and a poly adenelation signals; a selectable marker gene promoter; a ribosomal binding site; and an origin of replication. The synthetic or eukaryotic promoter, the nucleic acid sequence encoding the modified GHRH and the poly-adenylation signal comprise therapeutic elements of the synthetic mammalian expression plasmid. The therapeutic elements are operatively linked and located in a first operatively-linked arrangement. Similarly, the selectable marker gene promoter, the ribosomal binding site, the selectable marker gene sequence, and the origin of replication comprise replication elements of the synthetic mammalian expression plasmid; the replication elements are operatively linked and located in a second operatively-linked arrangement. The first-operatively-linked arrangement and the second-operatively-linked arrangement comprise a circular structure of the synthetic mammalian expression plasmid. In a preferred embodiment, the synthetic mammalian expression plasmid is utilized for plasmid mediated gene supplementation. Other preferred embodiments of a nucleic acid molecule that encodes a modified GHRH comprise a 3' untranslated region ("UTR") encompassing a portion of a human growth hormone 3'UTR, and a modified myogenic promoter (e.g. pSPc5 12).

In preferred embodiments, the nucleic acid molecule that encodes the modified GHRH is combined with a transfection-facilitating polypeptide (e.g. poly-L-glutamate) for delivering the composition into a muscle cell of a subject. The encoded modified GHRH is expressed in a tissue specific manner in the subject.

### BRIEF DESCRIPTION OF FIGURES

Figure 1 shows the amino acid sequence for porcine wild type growth hormone releasing hormone ("GHRH") and a protease resistant TI-GHRH analog with extended activity that can increase GH secretory activity and stability;

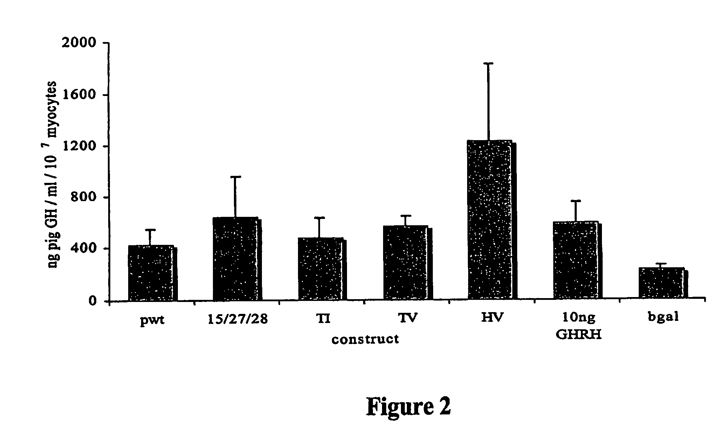

Figure 2 shows a the release of growth hormone ("GH") in porcine primary pituitary culture that were simulated by different GHRH species isolated from conditioned media of skeletal muscle cells transfected with myogenic expression vectors driving porcine GHRH analogs; the analogs are denoted as follows: porcine wild type GHRH (1-40)OH ("pwt"); pGHRH with amino acid substitutions of Gly15 to Ala, Met27 to Leu and Ser28 to Asn is represented by ("15/27/28"); the 15/27/28 construct plus the conversion of Ala2 to Ile2 is represented by ("TI-GHRH"); the 15/27/28 construct plus the conversion of Ala2 to Val2 is represented by ("TV-GHRH"); the 15/27/28 construct plus conversion of Tyr1 with His, and Ala2 with Val is represented by ("HV-GHRH"); a construct coding for *E.coli* beta-galactosidase("βgal") is used as a negative control; and a positive control of recombinant human ("10ng GHRH");

Figure 3 shows the enhanced stability of the TI-GHRH compared with wild type porcine GHRH over a 6 hour incubation in plasma;

Figure 4 shows a schematic representation of three plasmid constructs: the porcine wild type ("GHRH"); the TI-GHRH; and the β-galactosidase construct; all contain the SPc5-12 synthetic promoter and the 3' untranslated region ("UTR") of growth hormone ("GH"), kan (kanamycin resistance gene for bacterial selection), and neo (neomycin resistance gene for *in vivo* selection);

Figure 5 shows the relative levels of serum IGF-I concentration in pSP-GHRH injected mice versus placebo injected mice that were exposed to a single injection of any one of the super analog GHRH myogenic expression vectors;

Figure 6 shows the E peak early filling velocity of mice heart after TI-GHRH analogs were injected intra-muscularly compared with controls. Heart rate and A peak velocity are shown as control measures.

### DETAILED DESCRIPTION OF PREFERRED EMBODIMENTS

*Terms:*

The term "analog" as used herein includes any mutant of GHRH, or synthetic or naturally occurring peptide fragments of GHRH.

The term "codon" as used herein refers to any group of three consecutive nucleotide bases in a given messenger RNA molecule, or coding strand of DNA that specifies a particular amino-acid, or a starting or stopping signal for translation. The term codon also refers to base triplets in a DNA strand.

The term "coding region" as used herein refers to any portion of the DNA sequence that is transcribed into messenger RNA (mRNA) and then translated into a sequence of amino acids characteristic of a specific polypeptide.

The term "delivery" as used herein is defined as a means of introducing a material into a subject, a cell or any recipient, by means of chemical or biological process, injection, mixing, electroporation, sonoporation, or combination thereof, either without or under pressure.

The term "encoded GHRH" as used herein is a biologically active polypeptide.

The term "growth hormone" ("GH") as used herein is defined as a hormone that relates to growth and acts as a chemical messenger to exert its action on a target cell.

The term "growth hormone releasing hormone" ("GHRH") as used herein is defined as a hormone that facilitates or stimulates release of growth hormone, and to a lesser extent other pituitary hormones, such as prolactin.

The term "heterologous nucleic acid sequence" as used herein is defined as a DNA sequence consisting of differing regulatory and expression elements.

The term "modified GHRH" as used herein is a polypeptide that has been engineered to contain an amino acid sequence that is distinct from the wild-type GHRH polypeptide while simultaneously having similar or improved biologically activity when compared to the wild-type GHRH polypeptide. The wild-type GHRH polypeptide is the naturally occurring species-specific GHRH polypeptide of a subject, a cell or any recipient of the modified GHRH.

The term "nucleic acid expression construct" as used herein refers to any type of genetic construct comprising a nucleic acid coding for a RNA capable of being transcribed. The transcribed RNA is then capable of being translated into a peptide, polypeptide, or protein. The term "expression vector" or "expression plasmid" can also be used interchangeably.

The term "subject" as used herein refers to any species of the animal kingdom. In preferred embodiments it refers more specifically to humans and domesticated animals.

The term "domesticated animal" as used herein refers to animals used for: pets (e.g. cats, dogs, etc.); work (e.g. horses, cows, etc.); food (chicken, fish, lambs, pigs, etc); and all others known in the art.

The term "operatively linked" as used herein refers to elements or structures in a nucleic acid sequence that are linked by operative ability and not physical location. The elements or structures are capable of, or characterized by accomplishing a desired operation. It is recognized by one of ordinary skill in the art that it is not necessary for elements or structures in a nucleic acid sequence to be in a tandem or adjacent order to be operatively linked.

The term "promoter" as used herein refers to a sequence of DNA that directs the transcription of a gene. A promoter may direct the transcription of a prokaryotic or eukaryotic gene. A promoter may be "inducible," initiating transcription in response to an inducing agent or, in contrast, a promoter may be "constitutive," whereby an inducing agent does not regulate the rate of transcription. A promoter may be regulated in a tissue-specific or tissue-preferred manner, such that it is only active in transcribing the operable linked coding region in a specific tissue type or types.

The term "replication element" as used herein comprises nucleic acid sequences that will lead to replication of a plasmid in a specified host. One skilled in the art of molecular biology will recognize that the replication element may include, but is not limited to, a selectable marker gene promoter, a ribosomal binding site, a selectable marker gene sequence, and an origin of replication.

The term "therapeutic element" as used herein comprises nucleic acid sequences that will lead to an *in vivo* expression of an encoded gene product. One skilled in the art of molecular biology will recognize that the therapeutic element may include, but is not limited to a promoter sequence, a poly [A] sequence, or a 3' or 5' UTR.

The term "vector" as used herein refers to any vehicle that delivers a nucleic acid into a cell or organism. Examples include plasmid vectors, viral vectors, liposomes, or cationic lipids.

The standard one and three letter abbreviations for amino acids used herein are as follows: Alanine, A ala; Arginine, R, arg; Asparagine, N, asn; Aspartic acid, D, asp; Cysteine, C, cys; Glutamine, Q, gln; Glutamic acid, E, glu; Glycine, G, gly; Histidine, H, his; Isoleucine, I, ile; Leucine, L, leu; Lysine, K, lys; Methionine, M, met; Phenylalanine, F, phe; Proline, P, pro; Serine, S, ser; Threonine, T, thr; Tryptophan, W, trp; Tyrosine, Y, tyr; Valine, V, val.

In a preferred embodiment, the nucleic acid construct or vector of the present invention is a plasmid which comprises a synthetic myogenic (muscle-specific) promoter, a synthetic nucleotide sequence encoding a modified growth hormone releasing hormone (GHRH) or its analog, and a 3' untranslated region (3'UTR).

**Promoters and Enhancers.** A "promoter" is a control sequence that is a region of a nucleic acid sequence at which the initiation and rate of transcription are controlled. It may contain genetic elements where regulatory proteins and molecules may bind such as RNA polymerase and transcription factors. The phrases "operatively positioned," "operatively linked," "under control," and "under transcriptional control" mean that a promoter is in a correct functional location and/or orientation in relation to a nucleic acid sequence to control transcriptional initiation and/or expression of that sequence. A promoter may or may not be used in conjunction with an "enhancer," which refers to a cis-acting regulatory sequence involved in the transcriptional activation of a nucleic acid sequence.

A promoter may be one of naturally-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other prokaryotic, viral, or eukaryotic cell, and promoters or enhancers not "naturally occurring," *i.e.*, containing different elements of different transcriptional regulatory regions, and/or mutations that alter expression. In addition to producing nucleic acid sequences of promoters and enhancers synthetically, sequences may be produced using recombinant cloning and/or nucleic acid amplification technology, including PCR™. Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the cell type, organelle, and organism chosen for expression. Those of skill in the art of molecular biology generally know the use of promoters, enhancers, and cell type combinations for protein expression. The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous. In a specific embodiment the promoter is a synthetic myogenic promoter.

The identity of tissue-specific promoters or elements, as well as assays to characterize their activity, is well known to those of skill in the art. Examples of such regions include the human LIMK2 gene, the somatostatin receptor 2 gene, murine epididymal retinoic acid-binding gene, human CD4, mouse alpha2 (XI) collagen, D1A dopamine receptor gene, insulin-like growth factor II, human platelet endothelial cell adhesion molecule-1.

**Initiation Signals and Internal Ribosome Binding Sites.** A specific initiation signal also may be required for efficient translation (synthesis of the encoded protein) of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments of the invention, the use of internal ribosome entry sites ("IRES") elements are used to create multigene, or polycistronic messages. IRES elements are able to bypass the ribosome scanning model of 5' methylated Cap-dependent translation and begin translation at internal sites. IRES elements from two members of the picornavirus family (polio and encephalomyocarditis) have been described, as well an IRES from a mammalian message. IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message.

**Multiple Cloning Sites.** Vectors can include a multiple cloning site ("MCS'), which is a nucleic acid region that contains multiple restriction enzyme sites, any of which can be used in conjunction with standard recombinant technology to digest the vector. "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

**Splicing Sites.**Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression.

**Polyadenylation Signals.** In expression, one will typically include a polyadenylation signal to effect proper polyadenylation of the transcript. The nature of the polyadenylation signal is not believed to be crucial to the successful practice of the invention, and/or any such sequence may be employed. Preferred embodiments include the bovine or human growth hormone polyadenylation signal, convenient and/or known to function well in various target cells. Also contemplated as an element of the expression cassette is a transcriptional termination site. These elements can serve to enhance message levels and/or to minimize read through from the cassette into other sequences.

**Origins of Replication.** In order to propagate a vector in a host cell, it may contain one or more origins of replication sites (often termed "ori"), which is a specific nucleic acid sequence at which replication is initiated. Alternatively an autonomously replicating sequence (ARS) can be employed if the host cell is yeast.

**Selectable and Screenable Markers.** In certain embodiments of the invention, the cells that contain the nucleic acid construct of the present invention may be identified *in vitro* or *in vivo* by including a marker in the expression vector. Such markers would confer an identifiable change to the cell permitting easy identification of cells containing the expression vector. Generally, a selectable marker is one that confers a property that allows for selection. A positive selectable marker is one in which the presence of the marker allows for its selection, while a negative selectable marker is one in which its presence prevents its selection. An example of a positive selectable marker is a drug resistance marker, such as the antibiotic resistance gene on the plasmid constructs (such as kanamycin, ampicylin, gentamycin, tetracycline, or chloramphenicol).

Usually the inclusion of a drug selection marker aids in the cloning and identification of transformants, for example, genes that confer resistance to neomycin, puromycin, hygromycin, DHFR, GPT, zeocin and histidinol are useful selectable markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selectable and screenable markers are well known to one of skill in the art.

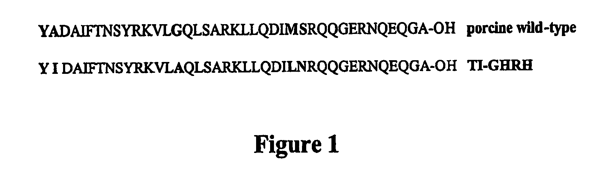

**Growth hormone releasing hormone:** Growth hormone releasing hormone ("GHRH") has a short half-life in the circulatory system, both in humans and pigs (Frohman et al., 1984). However, by employing GHRH analogs with a prolonged biological half-life and/or improved secretagogue activity, it was possible to achieve enhanced growth hormone ("GH") secretion. Therefore, GHRH mutants were generated by site-directed mutagenesis of a porcine (1-40)OH form of the cDNA (Seq ID #1). The site directed mutagenesis altered several amino acid codons of the wild type porcine GHRH (Seq ID #2). TI-GHRH mutant composition is shown in Figure 1 and (Seq ID #3). The substitution of Gly15 to Ala15 was used to increase α-helical conformation and amphiphilic structure that resulted in decreased cleavage by trypsin-like enzymes (Su et al., 1991). Also, GHRH analogs with the Ala15-substitution display a 4-5 times higher affinity for the GHRH receptor (Reiss et al., 1993). We substituted Met27, Ser28 with Leu27, Asn28 (Kubiak et al., 1989) in order to reduce loss of biological activity due to oxidation of the Met27, thus forming a triple amino acid substitution denoted as 15/27/28-GHRH (Seq ID #4). Dipeptidyl peptidase IV is the prime serum GHRH degradative enzyme(Martin et al., 1993) . Lower affinity dipeptidase substrates were created by further substitutions of 15/27/28-GHRH, and converting Ala2 for Ile2 (TI-GHRH, T1I2A15L27N28) or for Val2 ('TV-GHRH - Seq ID #5) or by converting Tyr1 and Ala2 with His1 and Val2 (HV-GHRH - Seq ID #6). The HV-GHRH super-analog was presented in the U.S. Patent Application S.N. 10/021,403 filed on December 12, 2001 and titled "Administration of nuclic acid sequence to female animal to enhance growth in offspring" with Schwartz, et al., listed as inventors, and U.S. Patent Application S.N. 09/624,268 filed on July 24, 2000, 2000 and titled "Super Active Porcine Growth Hormone Releasing Hormone Analog" with Schwartz, et al., listed as inventors.

The invention may be better understood with reference to the following examples, which are representative of some of the embodiments of the invention, and are not intended to limit the invention.

### EXAMPLE 1

To test the biological potency of the mutated porcine GHRH (pGHRH) cDNA, a plasmid vector was engineered that could direct very high levels of skeletal muscle-specific gene expression with, the use of a newly described synthetic muscle promoter, SPc5-12 (Li et al., 1999).A 228 -bp fragment of pGHRH, which encodes the 31 amino acid signal peptide and a mature peptide pGHRH (Tyrl-Gly40) and/or the modified GHRH, or functional biological equivalents thereof, followed by the 3' untranslated region of the human GH ("hGH") cDNA. All expression vector elements were operatively linked and incorporated into myogenic GHRH expression vectors. Skeletal myoblasts were transfected with each construct. Purified GHRH moieties from conditioned culture media were assayed for potency by their ability to induce GH secretion in pig anterior pituitary cell cultures. As shown in Figure 2, media were collected from the pituitary cell cultures after 24 hours and analyzed for porcine-specific GH by radioimmunoassay. The modified GHRH species (15/27/28-GHRH,; TI-GHRH; TV-GHRH, HV-GHRH) showed 20% to 50% improvements in their capacity to stimulate GH secretion compared to wild-type porcine GHRH ("wt-GHRH"), as indicated by the increase in porcine GH levels from a baseline value of 200ng/ml to 1600ng/ml. Although not wanting to be bound by theory, the increase was probably produced by an increased affinity for the GHRH receptors present on the pituitary cells.

Experimental Cell culture conditions were as follows: The Minimal Essential Medium ("MEM"), heat-inactivated horse serum ("HIHS"), gentamycin, Hanks Balanced Salt Solution ("HBSS"), lipofectamine were obtained from Gibco BRL (Grand Island, NY). Primary chicken myoblast cultures were obtained and transfected as previously described (Bergsma et al., 1986; Draghia-Akli et al., 1997). After transfection, the medium was changed to MEM which contained 2% HIHS to allow the cells to differentiate. Media and cells were harvested 72 hours post-differentiation. One day before harvesting, cells were washed twice in HBSS and the media changed to MEM, 0.1% bovine serum albumin ("BSA"). Conditioned media was treated by adding 0.25 volume of 1% triflouroacetic acid ("TFA") and 1mM phenylmethylsulfonylflouride ("PMSF"), frozen at -80°C, lyophilized, purified on C-18 Sep-Columns (Peninsula Laboratories, Belmont, CA), relyophilized and used in the radioimmuno assay ("RIA") or resuspended in media conditioned for primary pig anterior pituitary culture. The pig anterior pituitary culture was obtained as previously described (Tanner et al., 1990).

Plasmid vectors containing the muscle specific synthetic promoter SPc5-12 were previously described (Li et al., 1999). Wild type and mutated porcine GHRH cDNAs were generated by site directed mutagenesis of GHRH cDNA (Altered Sites II *in vitro* Mutagenesis System, Promega, Madison, WI), and cloned into the BamHI/ Hind III sites of pSPc5-12, to generate pSP-wt GHRH, or pSP-TI-GHRH respectively. The 3' untranslated region (3'UTR) of growth hormone was cloned downstream of GHRH cDNA. The resultant plasmids contained mutated coding region for GHRH, and the resultant amino acid sequences were not naturally present in mammals. Although not wanting to be bound by theory, the effects on treating GH deficient diseases is determined ultimately by the circulating levels of needed hormones. Several different plasmids that encode modified GHRH or functional biological equivalent thereof are as follows:

| **Plasmid** | **Encoded Amino Acid Sequence** |
|---|---|
| **wt-GHRH** | **YA**DAIFINSYRKVL**G**QLSARKLLQDI**MSR**QQGERNQEQGA-OH |
| **HV-GHRH** | **HV**DAIFTNSYRKVL**A**QLSARKLLQDI**LN**RQQGERNQEQGA-OH |
| **TI-GHRH** | **YI**DAIFTNSYRKVL**A**QLSARKLLQDI**LN**RQQGERNQEQGA-OH |
| **TV-GHRH** | **YV**DAIFTNSYRKVL**A**QLSARKLLQDI**LN**RQQGERNQEQGA-OH |
| 15/27/28**GHRI** | **YA**DAIFTNSYRKVL**A**QLSARKLLQDI**LN**RQQGERNQEQGA-OH |

In general, the encoded GHRH or functional biological equivalent thereof is of formula:

-**A₁**-**A₂**DAIFTNSYRKVL-**A₃**-QLSARKLLQDI-**A₄**-**A₅**RQQGERNQEQGA-OH

wherein: **A₁** is a D-or L-isomer of an amino acid selected from the group consisting of tyrosine ("Y"), or histidine ("H"); **A₂** is a D-or L-isomer of an amino acid selected from the group consisting of alanine ("**A**"), valine ("V"), or isoleucine ("I"); **A₃** is a D-or L-isomer of an amino acid selected from the group consisting of alanine ("**A**") or glycine ("G"); **A₄** is a D-or L-isomer of an amino acid selected from the group consisting of methionine ("M"), or leucine ("L"); **A₅** is a D-or L-isomer of an amino acid selected from the group consisting of serine ("S") or asparagine ("N").

Another plasmid that was utilized included the pSP-SEAP construct that contains the SacI/ HindIII SPc5-12 fragment, secreted embryonic alkaline phosphatase (SEAP) gene and SV40 3'UTR from pSEAP-2 Basic Vector (Clontech Laboratories, Inc., Palo Alto, CA).

The plasmids described above do not contain polylinker, IGF-I gene, a skeletal alpha-actin promoter or a skeletal alpha actin 3' UTR /NCR. Furthermore, these plasmids were introduced by muscle injection, followed by *in vivo* electroporation, as described below.

### EXAMPLE 2

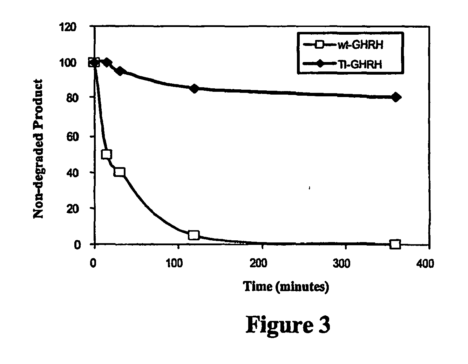

Stability of wild type GHRH and the analog TI-GHRH was then tested in porcine plasma by incubation of GHRH peptides in plasma, followed by solid phase extraction and HPLC analysis (Su et al., 1991). As shown in Figure 3, at least 60% of the pGHRH was degraded within 30 minutes of incubation in plasma. In contrast, incubation of TI-GHRH in pig plasma for up to 6 hours showed that at least 70% of the polypeptide was protected against enzymatic cleavage indicating a considerable increase in the resistance of TI-GHRH to serum protease activity. No degradation was seen in the first 30 minutes of incubation. *Plasma proteolytic activity on GHRH molecules.* Chemically synthesized TI-GHRH was prepared by peptide synthesis. Briefly, pooled porcine plasma was collected from control pigs, and stored at -80°C. At the time of the test, the porcine plasma was thawed, centrifuged and allowed to equilibrate at 37°C. Mutant and wild-type GHRH samples were dissolved in the plasma sample to a final concentration of 300µg/ml. Immediately after the addition of the GHRH, and 15, 30, 120 and 240 minutes later, 1mL of plasma was withdrawn and acidified with 1mL of 1M TFA. Acidified plasma was purified on C-18 affinity SEP-Pak columns, lyophilized, and analyzed by HPLC, using a Waters 600 multi-system delivery system, a Walters intelligent sample processor, type 717 and a Waters spectromonitor 490 (Walters Associates, Millipore Corp., Milford, MA). The mobile phase was (A) 0.1%TFA in H₂O, (B) 0.1%TFA in 95% ACN and 5% H₂O; the gradient was 80% (B) in 30 minutes. The flow rate was 0.75mL/min. Detection was performed at 214nm. The percent of peptide degraded at these time points was measured by integrated peak measurements.

### EXAMPLE 3

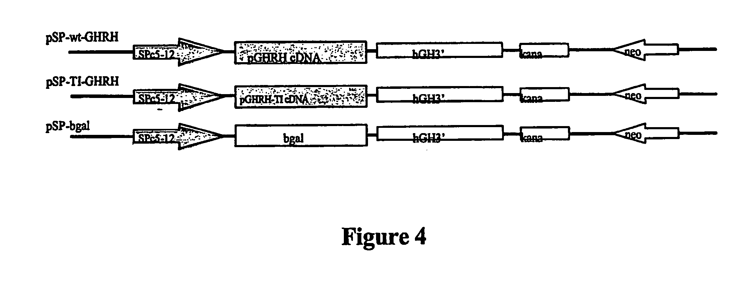

**Biological activity of TI-GHRH** in young animals. Muscle injection of pSP-TI-GHRH increases IGF-I serum levels over two months in treated mice. We asked if the optimized protease resistant pSP-TI-GHRH vector could affect *in vivo,* long-term expression of GHRH and stimulate secretion of GH and subsequently, IGF-I. Schematic maps of pSP-TI-GHRH, the wild type construct, pSP-wt GHRH (positive control), and an *E.coli.* β-galactosidase expression vector, pSP-βal (placebo control), are shown in Figure 4. Five weeks old SCID mice (immuno-deficient mice) were injected into the tibialis anterior muscle with 7.5 micrograms of one of the constructs. The injected muscle was placed within a caliper and electroporated, using optimized conditions of 200V/cm with 6 pulses of 60 milliseconds, as described in Materials and Methods (Aihara and Miyazaki, 1998).

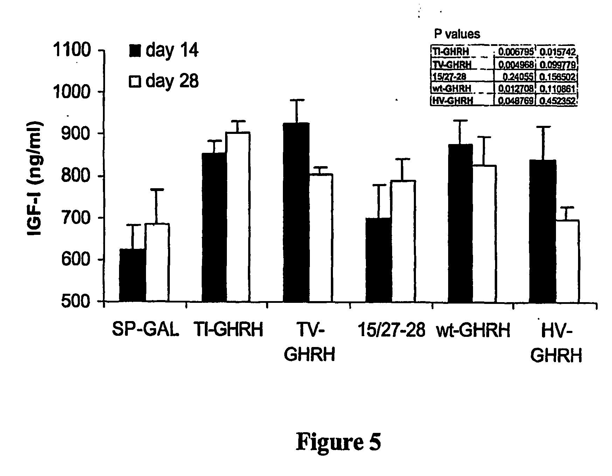

Animals were bled up to two month post-injection and serum was used for IGF-I measurements (Figure 5). At 14 and 28 days post-injection, blood was collected and IGF-I levels were measured. All GHRH plasmid injected groups had highly significant increases in IGF-I levels compared to control animals, p < 0.005. Some groups developed neutralizing antibodies, and in these cases the IGF-I levels dropped by the second time point. The animals injected with TI-GHRH did not develop any antibodies, and their modified TI-GHRH expression continued for two month, and correlated with significant changes in their body composition.

### Example 4

A long-lasting therapy has the potential to replace classical GH therapy regimens and may stimulate the GH axis in a more physiologically appropriate manner. It is known that GHRH stimulates bone formation (Dubreuil et al., 1996), and our therapy may be used to promote post-fracture bone growth. Data show that GH plus IGF-I (delivered as recombinant proteins) synergistically increase lean muscle and body weight, total body weight, and were more effective in re-epithelialization of a bum wound than either GH or IGF-I alone (Meyer et al., 1996). Studies also showed that long-term stimulation of the GH axis, which includes doses in the range given to humans during clinical trials of GH deficiency and to revert age-related physiologic declines, has no overt deleterious effects on longevity and pathology in aged rodents (Kalu et al., 1998).

**Using modified GHRH** it was possible to show that body composition was altered in treated animals. For example, twenty nine ("29") month old mice were injected into the tibialis anterior muscle with 15 micrograms TI-GHRH plasmid. The control group received the pSP-βgal construct previously described. Injection followed by electroporation with calipers with standard conditions 200V/cm, gap 5 mm on average, 3 pulses x 2, 50 milliseconds/pulse. Cardiac function in these animals was evaluated by Doppler at day 0, 10 and 20 and echocardiogram at day 20. At the end of the experiment, body composition was performed post-mortem.

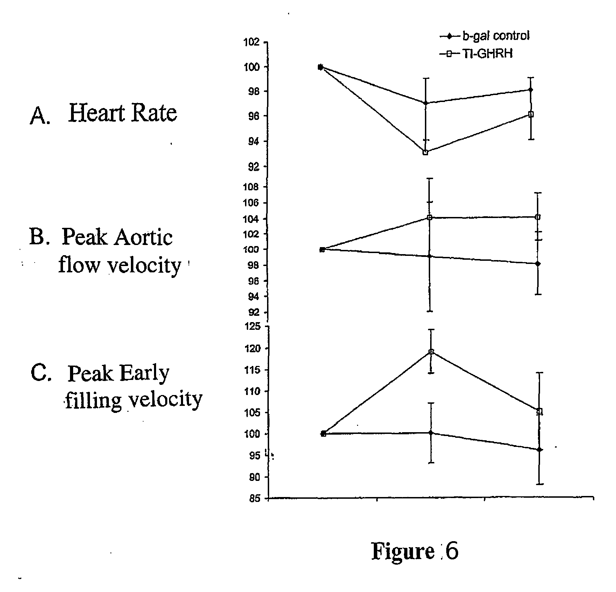

TI-GHRH treated animals had increased cardiac function, as assessed by Doppler and echocardiogram. Heart rate is stable (within 5% throughout the assay) (Figure 6A). Peak aortic flow velocity does not change either (Figure 6B). This is unexpected as GH and IGF-I are supposed to induce eNOS and reduce peripheral vascular resistance. Altered diastolic filling is an important contributor to several cardiovascular disorders (Houlind et al., 2002; Richartz et al., 2002; Tang et al., 2002). Peak early filling velocity, a measure of the cardiac diastolic filling indices, increases impressively by 20% at 10 days post-treatment (Figure 6C).

Among the non-viral techniques for plasmid transfer *in vivo,* the direct injection of plasmid DNA into muscle is simple, inexpensive, and safe. Applications of this methodology have been limited by the relatively low expression levels of the transferred DNA expression vectors. Previously, these levels have been insufficient to ensure systemic physiological levels of secreted proteins such as hormones, neurotrophic factors or coagulation factors in large mammals. Although not wanting to be bound by theory, in order to obtain growth of a large mammal by plasmid therapy, it is necessary to increase the potency of the myogenic vector system. The inventors recently described (Li et al., 1999) a strategy for the construction and the characterization of novel muscle synthetic promoters by the random assembly of E-boxes, MEF-2, TEF-1 and SRE sites. Several synthetic muscle promoters were identified whose transcriptional activity in terminally differentiated muscle greatly exceeded that of the natural myogenic skeletal α-actin gene promoter and viral promoters. Analysis of direct intramuscular injection of SPc5-12 driven DNA plasmid in normal mouse muscle revealed a 6-8-fold increase in activity over the ubiquitously expressed CMV promoter even after a month. As shown in figure 2, SPc5-12 was capable of eliciting moderate increases in growth and IGF-I levels by driving GHRH production in animals. Severe combined immunodeficient (SCID) adult male mice (aged 5-6 weeks at the beginning of the experiment) or. NIH C57/B16 mice (aged 29 month) were housed and cared for in the animal facility of Baylor College of Medicine, Houston, TX. Animals were maintained under environmental conditions of 10h light / 14h darkness, in accordance with NIH Guidelines, USDA and Animal Welfare Act guidelines, and the protocol was approved by the Institutional Animal Care and Use Committee. On day 0, the animals were weighed and then, the left tibialis anterior muscle of mice was injected with plasmids in 25 µl PBS. The injection was followed by caliper electroporation, as previously described (Draghia-Akli et al., 1999). The animals were bled periodically, and serum was used to measure IGF-I levels. At the end of the experiment, body composition was performed *in vivo,* using the DEXA technique, and than at necropsy. Blood was collected, centrifuged immediately at 4°C, and stored at - 80°C prior to analysis. Organs, carcass, and fat from injected animals and controls were removed, weighed and snap frozen in liquid nitrogen.

Mouse IGF-I Radioimmunoassay: Mouse IGF-I was measured by heterologous, 100% cross-reacting rat radioimmunoassay. The sensitivity of the assay was 0.8 ng/ml; intra-assay and inter-assay variation was 3.4% and 4.5% respectively. The statistics and values shown in the figures are the mean ± s.e.m. Specific p values were obtained by comparison using Students t-test or ANOVA analysis. A p<0.05 was set as the level of statistical significance.

Another significant improvement of our plasmid vector was the employment of a GHRH analog, TI-GHRH. Some individual amino acid substitutions leading to protease resistant GHRH molecules were previously tested in farm animals and humans (Frohman et al., 1989; Martin et al., 1993). The inventors have found that novel combination of five amino acid substitutions in the TI-GHRH construct resulted in increased GH secretagogue activity (as shown in assays on pig anterior pituitary somatotrophic cells) and was more resistant to serum proteases *in vivo* (Figure 2).

### EXAMPLE 5

Although not wanting to be bound by theory, electro-plasmid therapy allows genes to be efficiently transferred and expressed in desired organs or tissues, and it may represent a new approach for highly effective plasmid supplementation therapy, that does not require viral genes or particles. The electroporation system was used previously in rodents and small animals and does not appear to cause significant distress. Electro-plasmid therapy increased transfection efficiency over 100-fold compared to classical plasmid therapy techniques and allowed for prolonged TI-GHRH expression.

Although not wanting to be bound by theory, enhanced biological potency and enhanced delivery protocols reduces the theoretical quantity of GHRH plasmid needed to achieve physiological levels of GH production and secretion. Treated mice did not experience any side effects from the therapy, had normal biochemical profiles, and with no associated pathology. The profound increases in IGF-I levels growth enhancement and improved cardiac function indicate that ectopic expression of myogenic TI-GHRH vectors has the potential to replace classical GH therapy regimens and may stimulate the GH axis in a more physiologically appropriate manner. The TI-GHRH molecule, which displays a high degree of stability and GH secretory activity, may also be useful in human clinical medicine, since the serum proteases that degrade GHRH are similar in most mammals.

Hormones (e.g. GHRH and GH) often contain a complex feedback-regulated pathway, which are further complicated by chronic conditions such as cancer or AIDS. Without direct experimentation of GHRH or biological equivalents used in plasmid mediated supplementation, a beneficial therapy could not have been predicated by one skilled in the art to determine which modified GHRH encoded sequences will yield desired results. The invention described herein contains the compositions, descriptions, and results of essential experimentation that explored tissue specific and inducible regulation of distinctive nucleic acid sequences that encoded modified GHRH which was not obvious based upon prior art.

One skilled in the art readily appreciates that the disclosed invention is well adapted to carry out the mentioned and inherent objectives. Growth hormone, growth hormone releasing hormone, modified growth hormone releasing hormone, plasmids, vectors, pharmaceutical compositions, treatments, methods, procedures and techniques described herein are presented as representative of the preferred embodiments and are not intended as limitations.

### REFERENCES CITED

**U.S. PATENT DOCUMENTS**
   U.S. 5,847,066, U.S. 3,846,936, U.S. 5,792,747, U.S. 5,776,901, U.S. 5,756,264, U.S. 5,696,089, U.S. 5,486.505, U.S. 5,137,872, U.S. 5,084,442, U.S. 5,036,045, U.S. 5,023,322, U.S. 4,839,344, U.S. 4,410,512, U.S. RE33,699, U.S. 4,833,166, U.S. 4,228,158, U.S. 4,228,156, U.S. 4,226,857, U.S. 4,224,316, U.S. 4,223,021, U.S. 4,223,020, U.S. 4,223,019.
**PUBLICATION REFERENCE LIST**
   Abboud, S. L., et al. 1991. J Clin. Invest 88: 470-475.
   Acsadi, G., et al. 1991. Nature 352: 815-818.
   Aihara, H. and J. Miyazaki. 1998. Nat. Biotechnol. 16: 867-870.
   Auernhammer, C. J. and C. J. Strasburger. 1995. European Journal of Endocrinology 133: 635-645.
   Bergsma, D. J., et al. 1986. Molecular & Cellular Biology 6: 2462-2475.
   Butler, A. A., et al 1994. Mol. Cell Endocrinol. 101: 321-330.
   Caroni, P. and C. Schneider- 1994.J. Neurosci. 14: 3378-3388.
   Clark, R. et al. 1993. J Clin. Invest 92: 540-548.
   Corpas, E. et al. 1993. Journal of Clinical Endocrinology & Metabolism 76: 134-138.
   Cottam, Y. H. et al. 1992. Endocrinology 130: 2924-2930.
   Danko, I. and J. A. Wolff. 1994. Vaccine 12: 1499.1502.
   Davis, H. L. et al. 1993. Human Gene Therapy 4; 151-159.
   Draghia-Akli, R et al. 1999. Nat. Biotechnol. 17: 1179-1183.
   Draghia-Akli. R et al. 1997. Nature biotechnology 15: 1285-1289.
   Draghia-Akli, R et al. 2002. FASEB J. 16: 426-428.
   Dubreuil, P. et al. 1996. Canadian Journal of Veterinary Research 60: 7-13.
   Etherton, T. D. et al. 1986. Journal of Animal Science 63 : 1389-1399.
   Foncea, R. et al. 1997. J. Biol. Chem. 272: 19115-19124.
   Frohman, L. A et al. 1992 Frontiers in Neuroendocrinology 13: 344-405.
   Frohman, L. A. et al. 1989. J. Clin. Invest. 83: 1533-1540.
   Frohman, L. A et al. 1984. J. Clin. Invest. 73: 1304-1311.
   Gesundheit. N. and J. K. Alexander. 1995. B. D. Weintraub (Ed.) Molecular Endocrinology: Basic Concepts and Clinical Correlations. pp. 491-507. Raven Press, Ltd., New York.
   Hoess, R. H. and K. Abremski. 1985. J. Mol. Biol. 181: 351-362.
   Houlind, K. et al. 2002. Magn Rcson. Imaging 20: 249-260.
   Jardieu, P. et al. 1994. J Immunol. 152: 4320- 4327.
   Kalu, D. N. et al, 1998. J. Garontol. A. Biol. Sci. Med. Sci. 53: H432-H463.
   Koo, G. C. et al. 2001. J Immunol. 166: 4195-4201.
   Kubiak, T. M. et al. 1989. Drug Metabolism & Disposition 17: 393-397.
   Landreth, K. S. et al. 1985. J Immunol. 134: 2305-2309.
   Landreth, K. S. et al. 1992. Blood 80: 1207-1212.
   Lapierre, H. et al. 1991. Journal of Animal Science 69: 587-598.
   LeRoith, D. et al. 1996. Endocrinology 137: 1071-1079.
   Li. X. et al. Nature biotechnology 17 [3]. 241-245.1999.
   Liu, J. L. and D. LeRoith. 1999. Endocrinology 140: 5179-5184.
   Lowe, W. L. et al. 1989. J. Clin. Invest 84: 619-626.
   Martin, R. A. et al. 1993. Biochimica et Biophysica Acta 1164: 252-260.
   Meyer, N. A. et al. 1996. J. Trauma 41 : 1008-1012.
   Muramatsu, T. et al. 1998. Int. J. Mol. Med. 1: 55-62.
   Parks, J. S. et al. 1995. B. D. Weintraub (Ed.) Molecular Endocrinology: Basic Concepts and Clinical Correlations. pp. 473-490. Raven Press, Ltd., New York.
   Parrizas, M. and D. LeRoith. 1997. Endocrinology 138: 1355-1358.
   Rabinovsky, E. D. et al. 1992. J. Neurosci. Res. 31: 188-192.
   Reiss, K. et al. 1993. Exp. Cell Res. 207: 348-360.
   Richartz, B. et al. 2002. Am. J. Cardiol. 90: 390.
   Robbins, K et al. 1994. Clin. Exp. Immunol. 95: 337-342.
   Su, C. M. et al. 1991. Hormone & Metabolic Research 23: 15-21.
   Tang, W. et al. 2002. Am. J. Hypertens. 15: 621-627.
   Tanner, J. W. et al. 1990. J. Endocrinol. 125: 109-115.
   Thorner, M. O. et al. 1984. Journal of Clinical Endocrinology & Metabolism 59: 846-849.
   Tripathy, S. K. et al. 1996. Proc. Natl. Acad. Sci. USA 93 : 10876-10880.
   Tsurumi, Y. et al. 1996. Circulation 94: 3281-3290.
   Valcavi, R. et al, 1995. Journal of Endocrinology & Metabolism 80:659-666
   van Rooij, E. M. et al. 2000. Vet. Immunol. Immunopathol. 74: 121-136.
   Veldhuis, J. D. et al. 1997. Endocrine 7: 41-48.
   Whang, L. et al. 1998. Endocrinology 139: 1354-1360.
   Wolff, J. A. et al. 1990. Science 247: 1465-1468.

## Claims

1. A composition comprising: a modified growth hormone releasing hormone (GHRH) with the general formula:
-A₁-A₂-DAIFTNSYRKVL-A₃-QLSARKLLQDI-A₄-A₅-RQQGERNQEQGA-OH
wherein: A₁ is a D-or L-isomer of the amino acid tyrosine (Y), or histidine (H); A₂ is a D-or L-isomer of the amino acid alanine (A), valine (V), or isoleucine (I); A₃ is a D-or L-isomer of the amino acid alanine (A) or glycine (G); A₄ is a D-or L-isomer of the amino acid methionine (M), or leucine (L); A₅ is a D-or L-isomer of the amino acid serine (S) or asparagine (N), for use as a medicament in improving cardiac function, as assessed by Doppler echocardiogram.

2. A nucleic acid expression construct encoding a modified growth hormone releasing hormone (GHRH) with the general formula:
-A₁-A₂-DAIFTNSYRKVL-A₃-QLSARKLLQDI-A₄-A₅-RQQGERNQEQGA-OH
wherein: A₁ is a D-or L-isomer of the amino acid tyrosine (Y), or histidine (H); A₂ is a D-or L-isomer of the amino acid alanine (A), valine (V), or isoleucine (I); A₃ is a D-or L-isomer of the amino acid alanine (A) or glycine (G); A₄ is a D-or L-isomer of the amino acid methionine (M), or leucine (L); A₅ is a D-or L-isomer of the amino acid serine (S) or asparagine (N), for use as a medicament in improving cardiac function, as assessed by Doppler echocardiogram.

3. The composition of claim 1 or a nucleic acid of claim 2, wherein the encoded modified GHRH is resistant to degradation when compared to the wild-type (wt) GHRH.

4. A composition of claim 1 comprising a modified GHRH of Seq ID No: 3.

5. The composition of claim 4, wherein the modified GHRH of Seq ID No: 3 is encoded by a nucleic acid expression construct.

6. A composition of claim 1, comprising a modified GHRH of Seq ID No: 4.

7. The composition of claim 6, wherein the modified GHRH of Seq ID No: 4 is encoded by a nucleic acid expression construct.

8. A composition of claim 1 comprising a modified GHRH of Seq ID No: 5.

9. The composition of claim 8, wherein the modified GHRH of Seq ID No: 5 is encoded by a nucleic acid expression construct.

10. The composition of any one of claims 5, 7 or 9, wherein the nucleic acid expression construct further comprises a transfection-facilitating polypeptide.

11. The composition of claim 10, wherein the transfection-facilitating polypeptide comprises a charged polypeptide.

12. The composition of claim 10, wherein the transfection-facilitating polypeptide comprises poly-L-glutamate.

13. The use of a nucleic acid expression construct encoding a modified growth hormone releasing hormone (GHRH) with the general formula:
-A₁-A₂-DAlFTNSYRKVL-A₃-QLSARKLLQDI-A₄-A₅-RQQGERNQEQGA-OH
wherein: A₁ is a D-or L-isomer of the amino acid tyrosine (Y), or histidine (H); A₂ is a D-or L-isomer of the amino acid alanine (A), valine (V), or isoleucine (I); A₃ is a D-or L-isomer of the amino acid alanine (A) or glycine (G); A₄ is a D-or L-isomer of the amino acid methionine (M), or leucine (L); A₅ is a D-or L-isomer of the amino acid serine (S) or asparagine (N) for the manufacture of a medicament for delivering into a cell of subject to improve cardiac function, as assessed by Doppler echocardiogram.

14. The use of claim 13, wherein delivering into the cell of the subject the nucleic acid expression construct is via electroporation.

15. The use of claim 13, wherein the cell of the subject is a somatic cell, a stem cell or a germ cell, wherein said germ cell is not a human germ cell.

16. The use of claim 13, wherein the cell of the subject is a muscle cell.

17. The use of claim 13, wherein the delivering into the cell of the subject the nucleic acid expression construct initiates expression of the encoded modified GHRH.

18. The use of claim 13, wherein the modified GHRH is expressed in a tissue specific cell of the subject.

19. The use of claim 13, wherein the modified GHRH is a biologically active polypeptide while simultaneously having similar or improved biologically activity when compared to the GHRH polypeptide.

20. The use of claim 13, wherein the modified GHRH is Seq ID No: 3.

21. The use of claim 13, wherein the modified GHRH is Seq ID No: 4.

22. The use of claim 13, wherein the modified GHRH is Seq ID No: 5.

23. The use of claim 13, wherein the modified GHRH is a biologically active polypeptide while simultaneously having similar or improved biologically activity when compared to a wild-type GHRH (wt-GHRH) polypeptide.

24. The use of claim 13, wherein the subject is a human or domesticated animal.

25. The use of claim 13, wherein the modified GHRH is resistant to degradation when compared to the wt-GHRH.

## Patentansprüche

1. Zusammensetzung umfassend ein modifiziertes Wachstumshormon freisetzendes Hormon (GHRH, *'growth hormone releasing hormone'*) mit der allgemeinen Formel:
-A₁-A₂-DAIFTNSYRKVL-A₃-QLSARKLLQDI -A₄-A₅-RQQGERNQEQGA-OH,
wobei: A₁ ein D- oder L-Isomer der Aminosäure Tyrosin (Y) oder Histidin (H) ist; A₂ ein D- oder L-Isomer der Aminosäure Alanin (A), Valin (V) oder Isoleucin (I) ist; A₃ ein D- oder L-Isomer der Aminosäure Alanin (A) oder Glycin (G) ist; A₄ ein D-oder L-Isomer der Aminosäure Methionin (M) oder Leucin (L) ist; A₅ ein D- oder L-Isomer der Aminosäure Serin (S) oder Asparagin (N) ist, zur Verwendung als Medikament zur Verbesserung der Herzfunktion, wie mittels einer Doppler-Echokardiographie bewertet.

2. Nukleinsäure-Expressionskonstrukt, das ein modifiziertes Wachstumshormon freisetzendes Hormon (GHRH) mit der allgemeinen Formel:
-A₁-A₂-DAIFTNSYRKVL-A₃-QLSARKLLQDI-A₄-A₅-RQQGERNQEQGA-OH, kodiert,
worin: A₁ ein D- oder L-Isomer der Aminosäure Tyrosin (Y) oder Histidin (H) ist; A₂ ein D- oder L-Isomer der Aminosäure Alanin (A), Valin (V) oder Isoleucin (I) ist; A₃ ein D- oder L-Isomer der Aminosäure Alanin (A) oder Glycin (G) ist; A₄ ein D- oder L-Isomer der Aminosäure Methionin (M) oder Leucin (L) ist; A₅ ein D- oder L-Isomer der Aminosäure Serin (S) oder Asparagin (N) ist, zur Verwendung als Medikament zur Verbesserung der Herzfunktion, wie mittels einer Doppler-Echokardiographie bewertet.

3. Zusammensetzung nach Anspruch 1 oder Nukleinsäure nach Anspruch 2, worin das kodierte modifizierte GHRH im Vergleich zu dem Wildtyp (wt)-GHRH resistent gegen Degradation ist.

4. Zusammensetzung nach Anspruch 1, umfassend ein modifiziertes GHRH gemäß SEQ ID NR: 3.

5. Zusammensetzung nach Anspruch 4, worin das modifizierte GHRH gemäß SEQ ID NR: 3 durch ein Nukleinsäure-Expressionskonstrukt kodiert ist.

6. Zusammensetzung nach Anspruch 1, umfassend ein modifiziertes GHRH gemäß SEQ ID NR: 4.

7. Zusammensetzung nach Anspruch 6, worin das modifizierte GHRH gemäß SEQ ID NR: 4 durch ein Nukleinsäure-Expressionskonstrukt kodiert ist.

8. Zusammensetzung nach Anspruch 1, umfassend ein modifiziertes GHRH gemäß SEQ ID NR: 5.

9. Zusammensetzung nach Anspruch 8, worin das modifizierte GHRH gemäß SEQ ID NR: 5 durch ein Nukleinsäure-Expressionskonstrukt kodiert ist.

10. Zusammensetzung nach einem der Ansprüche 5, 7 oder 9, worin das Nukleinsäure-Expressionskonstrukt des Weiteren ein die Transfektion förderndes Polypeptid umfasst.

11. Zusammensetzung nach Anspruch 10, worin das die Transfektion fördernde Polypeptid ein geladenes Polypeptid umfasst.

12. Zusammensetzung nach Anspruch 10, worin das die Transfektion fördernde Polypeptid Poly-L-Glutamat umfasst.

13. Verwendung eines Nukleinsäure-Expressionskonstrukts, das ein modifiziertes Wachstumshormon freisetzendes Hormon (GHRH) mit der allgemeinen Formel:
-A₁-A₂-DAIFTNSYRKVL-A₃-QLSARKLLQDI-A₄-A₅-RQQGERNQEQGA-OH, kodiert,
worin: A₁ ein D- oder L-Isomer der Aminosäure Tyrosin (Y) oder Histidin (H) ist; A₂ ein D- oder L-Isomer der Aminosäure Alanin (A), Valin (V) oder Isoleucin (I) ist; A₃ ein D- oder L-Isomer der Aminosäure Alanin (A) oder Glycin (G) ist; A₄ ein D- oder L-Isomer der Aminosäure Methionin (M) oder Leucin (L) ist; A₅ ein D- oder L-Isomer der Aminosäure Serin (S) oder Asparagin (N) ist, zur Herstellung eines Medikaments zur Verabreichung in eine Zelle eines Subjekts zur Verbesserung der Herzfunktion, wie mittels einer Doppler-Echokardiographie bewertet.

14. Verwendung nach Anspruch 13, wobei die Verabreichung des Nukleinsäure-Expressionskonstrukts in die Zelle des Subjekts mittels Elektroporation erfolgt.

15. Verwendung nach Anspruch 13, wobei es sich bei der Zelle des Subjekts um eine somatische Zelle, eine Stammzelle oder eine Keimzelle handelt, wobei die Keimzelle keine menschliche Keimzelle ist.

16. Verwendung nach Anspruch 13, wobei es sich bei der Zelle des Subjekts um eine Muskelzelle handelt.

17. Verwendung nach Anspruch 13, wobei die Verabreichung des Nukleinsäure-Expressionskonstrukts in die Zelle des Subjekts die Expression des kodierten modifizierten GHRH initiiert.

18. Verwendung nach Anspruch 13, wobei das modifizierte GHRH in einer gewebespezifischen Zelle des Subjekts exprimiert wird.

19. Verwendung nach Anspruch 13, wobei das modifizierte GHRH ein biologisch aktives Polypeptid ist und gleichzeitig im Vergleich zu dem GHRH-Polypeptid eine ähnliche oder verbesserte biologische Aktivität aufweist.

20. Verwendung nach Anspruch 13, wobei es sich bei dem modifizierten GHRH um SEQ ID NR: 3 handelt.

21. Verwendung nach Anspruch 13, wobei es sich bei dem modifizierten GHRH um SEQ ID NR: 4 handelt.

22. Verwendung nach Anspruch 13, wobei es sich bei dem modifizierten GHRH um SEQ ID NR: 5 handelt.

23. Verwendung nach Anspruch 13, wobei das modifizierte GHRH ein biologisch aktives Polypeptid ist und gleichzeitig im Vergleich zu einem Wildtyp (wt)-GHRH-Polypeptid eine ähnliche oder verbesserte biologische Aktivität aufweist.

24. Verwendung nach Anspruch 13, wobei das Subjekt ein Mensch oder ein domestiziertes Tier ist.

25. Verwendung nach Anspruch 13, wobei das modifizierte GHRH im Vergleich zu dem Wildtyp (wt)-GHRH resistent gegen Degradation ist.

## Revendications

1. Composition comprenant une hormone de libération de l'hormone de croissance modifiée (GHRH) de formule général :
-A₁-A₂-DAIFTNSYRKVL-A₃-QLSARKLLQDI-A₄-A₅-RQQGERNQEQGA-OH, dans laquelle A₁ est un isomère D ou L de l'acide aminé tyrosine (Y) ou histidine (H) ; A₂ est un isomère D ou L de l'acide aminé alanine (A), valine (V) ou isoleucine (I) ; A₃ est un isomère D ou L de l'acide aminé alanine (A) ou glycine (G) ; A₄ est un isomère D ou L de l'acide aminé méthionine (M) ou leucine (L) ; A₅ est un isomère D ou L de l'acide aminé sérine (S) ou asparagine (N) pour l'utilisation comme médicament destiné à améliorer la fonction cardiaque évaluée par un échocardiogramme Doppler.

2. Construction d'expression d'acide nucléique codant pour une hormone de libération de l'hormone de croissance modifiée (GHRH) de formule général :
A₁-A₂ -DAIFTNSYRKVL-A₃-QLSARKLLQDI-A₄-A₅-RQQGERNQEQGA-OH, dans laquelle A₁ est un isomère D ou L de l'acide aminé tyrosine (Y) ou histidine (H) ; A₂ est un isomère D ou L de l'acide aminé alanine (A), valine (V) ou isoleucine (I) ; A₃ est un isomère D ou L de l'acide aminé alanine (A) ou glycine (G) ; A₄ est un isomère D ou L de l'acide aminé méthionine (M) ou leucine (L) ; A₅ est un isomère D ou L de l'acide aminé sérine (S) ou asparagine (N) pour l'utilisation comme médicament destiné à améliorer la fonction cardiaque évaluée par un échocardiogramme Doppler.

3. Composition selon la revendication 1 ou acide nucléique selon la revendication 2, dans laquelle/ lequel la GHRH modifiée codée résiste à la dégradation par rapport à la GHRH de type sauvage (wt).

4. Composition selon la revendication 1, comprenant une GHRH modifiée de SEQ ID N° _{:} 3.

5. Composition selon la revendication 4, dans laquelle/lequel la GHRH modifiée de SEQ ID N° . 3 est codée par une construction d'expression d'acide nucléique.

6. Composition selon la revendication 1, comprenant une GHRH modifiée de SEQ ID N° _{:} 4.

7. Composition selon la revendication 6, dans laquelle la GHRH modifiée de SEQ ID N° _{:} 4 est codée par une construction d'expression d'acide nucléique.

8. Composition selon la revendication 1, comprenant une GHRH modifiée de SEQ ID N° : 5.

9. Composition selon la revendication 8, dans laquelle la GHRH modifiée de SEQ ID N° : 5 est codée par une construction d'expression d'acide nucléique.

10. Construction selon l'une quelconque des revendications 5, 7 ou 9, dans laquelle la construction d'expression d'acide nucléique comprend en outre un polypeptide facilitant la transfection.

11. Composition selon la revendication 10, dans laquelle le polypeptide facilitant la transfection comprend un polypeptide chargé.

12. Composition selon la revendication 10, dans laquelle le polypeptide facilitant la transfection comprend le poly-L-glutamate.

13. Utilisation d'une construction d'expression d'acide nucléique codant pour une hormone de libération de l'hormone de croissance modifiée de formule générale :
-A₁-A₂-DAIFTNSYRKVL-A₃-QLSARKLLQDI-A₄-A₅-RQQGERNQEQGA-OH, dans laquelle A₁ est un isomère D ou L de l'acide aminé tyrosine (Y) ou histidine (H) ; A₂ est un isomère D ou L de l'acide aminé alanine (A), valine (V) ou isoleucine (I) ; A₃ est un isomère D ou L de l'acide aminé alanine (A) ou glycine (G) ; A₄ est un isomère D ou L de l'acide aminé méthionine (M) ou leucine (L) ; A₅ est un isomère D ou L de l'acide aminé sérine (S) ou asparagine (N) pour la production d'un médicament à distribuer dans une cellule du sujet pour améliorer la fonction cardiaque évaluée par un échocardiogramme Doppler.

14. Utilisation selon la revendication 13, dans laquelle la distribution dans la cellule du sujet de la construction d'expression d'acide nucléique s'effectue par électroporation.

15. Utilisation selon la revendication 13, dans laquelle la cellule du sujet est une cellule somatique, une cellule souche ou une cellule germinale, ladite cellule germinale n'étant pas une cellule germinale humaine.

16. Utilisation selon la revendication 13, dans laquelle la cellule du sujet est une cellule musculaire.

17. Utilisation selon la revendication 13, dans laquelle la distribution dans la cellule du sujet de la construction d'expression d'acide nucléique initie l'expression de la GHRH modifiée codée.

18. Utilisation selon la revendication 13, dans laquelle la GHRH modifiée est exprimée dans une cellule spécifique d'un tissu du sujet.

19. Utilisation selon la revendication 13, dans laquelle la GHRH modifiée est un polypeptide biologiquement actif ayant simultanément une activité biologiquement similaire ou améliorée par rapport au polypeptide de GHRH.

20. Utilisation selon la revendication 13, dans laquelle la GHRH modifiée est SEQ ID N° : 3.

21. Utilisation selon la revendication 13, dans laquelle la GHRH modifiée est SEQ ID N° : 4.

22. Utilisation selon la revendication 13, dans laquelle la GHRH modifiée est SEQ ID N° : 5.

23. Utilisation selon la revendication 13, dans laquelle la GHRH modifiée est un polypeptide biologiquement actif ayant simultanément une activité biologiquement similaire ou améliorée par rapport à un polypeptide GHRH de type sauvage (wt-GHRH).

24. Utilisation selon la revendication 13, dans laquelle le sujet est un animal humain ou domestique.

25. Utilisation selon la revendication 13, dans laquelle la GHRH modifiée résiste à la dégradation par rapport à la wt-GHRH.


### SEQUENCE LISTING

<110> Advisys
   Baylor college of Medicine Baylor
<120> PROTEASE RESISTANT TI-GROWTH HORMONE RELEASING HORMONE ("GHRH")
<130> 108328.00067 - AVSI-0024
<160> 9
<170> PatentIn version 3.1
<210> 1
   <211> 219
   <212> DNA
   z<213> artificial sequence
<220>
   <223> This is the cDNA for Porcine growth hormone releasing hormone
<400> 1
   atggtgctct gggtgttctt ctttgtgatc ctcaccctca gcaacagctc ccactgctcc      60
   ccacctcccc ctttgaccct caggatgcgg cggcacgtag atgccatctt caccaacagc     120
   taccggaagg tgctggccca gctgtccgcc cgcaagctgc tccaggacat cctgaacagg     180
   cagcagggag agaggaacca agagcaagga gcataatga                            219
<210> 2
   <211> 40
   <212> PRT
   <213> artificial sequence
<220>
   <223> This is the amino acid sequence for procine growth hormone releas ing hormone.
<400> 2
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Ser Arg Gln Gln Gly
               20                  25                  30
   Glu Arg Asn Gln Glu Gln Gly Ala
           35                  40
<210> 3
   <211> 40
   <212> PRT
   <213> artificial sequence
<220>
   <223> This is a modified amino acid sequence for growth hormone releasi ng hormone (GHRH). alpha-helical confomation was increased by s ubstituting Cly15 to Ala15.
<400> 3
   Tyr Ile Asp Ala Ile Phe Thr Asn Ser Tyr Arg Lys Val Leu Ala Gln
   1               5                   10                  15
   Leu Ser Ala Arg Lys Leu Leu Gln Asp Ile Leu Asn Arg Gln Gln Gly
               20                  25                  30
   Glu Arg Asn Gln Glu Gln Gly Ala
           35                  40
<210> 4
   <211> 40
   <212> PRT
   <213> artificial sequence
<220>
   <223> This is a porcine growth hormone releasing hormone ("GHRH") that has the following substitutions: Met27, Ser28 with Leu27 and Asn2 8.
<400> 4
   Tyr Ala Asp Ala Ile Phe Thr Asn Ser Tyr Arg Lys Val Leu Ala Gln
   1               5                   10                  15
   Leu Ser Ala Arg Lys Leu Leu Gln Asp Ile Leu Asn Arg Gln Gln Gly
               20                  25                  30
   Glu Arg Asn Gln Glu Gln Gly Ala
           35                  40
<210> 5
   <211> 40
   <212> PRT
   <213> artificial sequence
<220>
   <223> This is a growth hormone releasing hormone that has a val2 substi tution for a Ile 2.
<400> 5
   Tyr Val Asp Ala Ile Phe Thr Asn Ser Tyr Arg Lys Val Leu Ala Gln
   1               5                   10                  15
   Leu Ser Ala Arg Lys Leu Leu Gln Asp Ile Leu Asn Arg Gln Gln Gly
               20                  25                  30
   Glu Arg Asn Gln Glu Gln Gly Ala
           35                  40
<210> 6
   <211> 40
   <212> PRT
   <213> artificial sequence
<220>
   <223> This is a growth hormone releasing hormone ("GHRH") with a His1 a nd va12 substituting the Try1 and Ala2.
<400> 6
   His Val Asp Ala Ile Phe Thr Asn Ser Tyr Arg Lys Val Leu Ala Gln
   1               5                   10                  15
   Leu Ser Ala Arg Lys Leu Leu Gln Asp Ile Leu Asn Arg Gln Gln Gly
               20                  25                  30
   Glu Arg Asn Gln Glu Gln Gly Ala
           35                  40
<210> 7
   <211> 3534
   <212> DNA
   <213> artificial sequence
<220>
   <223> This is the nucleic acid sequence for the operatively linked comp onents of the TI-GHRH plamsmid.
<400> 7
   gttgtaaaac gacggccagt gaattgtaat acgactcact atagggcgaa ttggagctcc 60
   accgcggtgg cggccgtccg ccctcggcac catcctcacg acacccaaat atggcgacgg 120
   gtgaggaatg gtggggagtt atttttagag cggtgaggaa ggtgggcagg cagcaggtgt 180
   tggcgctcta aaaataactc ccgggagtta tttttagagc ggaggaatgg tggacaccca 240
   aatatggcga cggttcctca cccgtcgcca tatttgggtg tccgccctcg gccggggccg 300
   cattcctggg ggccgggcgg tgctcccgcc cgcctcgata aaggctccgg gggccggcgg 360
   cggcccacga gctacccgga ggagcgggag gcgccaagct ctagaactag tggatcccaa 420
   ggcccaactc cccgaaccac tcagggtcct gtggacagct cacctagctg ccatggtgct 480
   ctgggtgttc ttctttgtga tcctcaccct cagcaacagc tcccactgct ccccacctcc 540
   ccctttgacc ctcaggatgc ggcggtatat cgatgccatc ttcaccaaca gctaccggaa 600
   ggtgctggcc cagctgtccg cccgcaagct gctccaggac atcctgaaca ggcagcaggg 660
   agagaggaac caagagcaag gagcataatg actgcaggaa ttcgatatca agcttatcgg 720
   ggtggcatcc ctgtgacccc tccccagtgc ctctcctggc cctggaagtt gccactccag 780
   tgcccaccag ccttgtccta ataaaattaa gttgcatcat tttgtctgac taggtgtcct 840
   tctataatat tatggggtgg aggggggtgg tatggagcaa ggggcaagtt gggaagacaa 900
   cctgtagggc ctgcggggtc tattgggaac caagctggag tgcagtggca caatcttggc 960
   tcactgcaat ctccgcctcc tgggttcaag cgattctcct gcctcagcct cccgagttgt 1020
   tgggattcca ggcatgcatg accaggctca gctaattttt gtttttttgg tagagacggg 1080
   gtttcaccat attggccagg ctggtctcca actcctaatc tcaggtgatc tacccacctt 1140
   ggcctcccaa attgctggga ttacaggcgt gaaccactgc tcccttccct gtccttctga 1200
   ttttaaaata actataccag caggaggacg tccagacaca gcataggcta cctggccatg 1260
   cccaaccggt gggacatttg agttgcttgc ttggcactgt cctctcatgc gttgggtcca 1320
   ctcagtagat gcctgttgaa ttcgataccg tcgacctcga ggggggggcc ggtaccagct 1380
   tttgttccct ttagtgaggg ttaatttcga gcttggcgta atcatggtca tagctgtttc 1440
   ctgtgtgaaa ttgttatccg ctcacaattc cacacaacat acgagccgga agcataaagt 1500
   gtaaagcctg gggtgcctaa tgagtgagct aactcacatt aattgcgttg cgctcactgc 1560
   ccgctttcca gtcgggaaac ctgtcgtgcc agctgcatta atgaatcggc caacgcgcgg 1620
   ggagaggcgg tttgcgtatt gggcgctctt ccgcttcctc gctcactgac tcgctgcgct 1680
   cggtcgttcg gctgcggcga gcggtatcag ctcactcaaa ggcggtaata cggttatcca 1740
   cagaatcagg ggataacgca ggaaagaaca tgtgagcaaa aggccagcaa aaggccagga 1800
   accgtaaaaa ggccgcgttg ctggcgtttt tccataggct ccgcccccct gacgagcatc 1860
   acaaaaatcg acgctcaagt cagaggtggc gaaacccgac aggactataa agataccagg 1920
   cgtttccccc tggaagctcc ctcgtgcgct ctcctgttcc gaccctgccg cttaccggat 1980
   acctgtccgc ctttctccct tcgggaagcg tggcgctttc tcatagctca cgctgtaggt 2040
   atctcagttc ggtgtaggtc gttcgctcca agctgggctg tgtgcacgaa ccccccgttc 2100
   agcccgaccg ctgcgcctta tccggtaact atcgtcttga gtccaacccg gtaagacacg 2160
   acttatcgcc actggcagca gccactggta acaggattag cagagcgagg tatgtaggcg 2220
   gtgctacaga gttcttgaag tggtggccta actacggcta cactagaaga acagtatttg 2280
   gtatctgcgc tctgctgaag ccagttacct tcggaaaaag agttggtagc tcttgatccg 2340
   gcaaacaaac caccgctggt agcggtggtt tttttgtttg caagcagcag attacgcgca 2400
   gaaaaaaagg atctcaagaa gatcctttga tcttttctac ggggtctgac gctcagaaga 2460
   actcgtcaag aaggcgatag aaggcgatgc gctgcgaatc gggagcggcg ataccgtaaa 2520
   gcacgaggaa gcggtcagcc cattcgccgc caagctcttc agcaatatca cgggtagcca 2580
   acgctatgtc ctgatagcgg tccgccacac ccagccggcc acagtcgatg aatccagaaa 2640
   agcggccatt ttccaccatg atattcggca agcaggcatc gccatgggtc acgacgagat 2700
   cctcgccgtc gggcatgcgc gccttgagcc tggcgaacag ttcggctggc gcgagcccct 2760
   gatgctcttc gtccagatca tcctgatcga caagaccggc ttccatccga gtacgtgctc 2820
   gctcgatgcg atgtttcgct tggtggtcga atgggcaggt agccggatca agcgtatgca 2880
   gccgccgcat tgcatcagcc atgatggata ctttctcggc aggagcaagg tgagatgaca 2940
   ggagatcctg ccccggcact tcgcccaata gcagccagtc ccttcccgct tcagtgacaa 3000
   cgtcgagcac agctgcgcaa ggaacgcccg tcgtggccag ccacgatagc cgcgctgcct 3060
   cgtcctgcag ttcattcagg gcaccggaca ggtcggtctt gacaaaaaga accgggcgcc 3120
   cctgcgctga cagccggaac acggcggcat cagagcagcc gattgtctgt tgtgcccagt 3180
   catagccgaa tagcctctcc acccaagcgg ccggagaacc tgcgtgcaat ccatcttgtt 3240
   caatcatgcg aaacgatcct catcctgtct cttgatcaga tcttgatccc ctgcgccatc 3300
   agatccttgg cggcaagaaa gccatccagt ttactttgca gggcttccca accttaccag 3360
   agggcgcccc agctggcaat tccggttcgc ttgctgtcca taaaaccgcc cagtctagca 3420
   actgttggga agggcgatcg gtgcgggcct cttcgctatt acgccagctg gcgaaagggg 3480
   gatgtgctgc aaggcgatta agttgggtaa cgccagggtt ttcccagtca cgac 3534
<210> 8
   <211> 3534
   <212> DNA
   <213> artificial sequence
<220>
   <223> This is the nucleic acid sequence for the operatively linked comp onents of the TV-GHRH plasmid.
<400> 8
   gttgtaaaac gacggccagt gaattgtaat acgactcact atagggcgaa ttggagctcc 60
   accgcggtgg cggccgtccg ccctcggcac catcctcacg acacccaaat atggcgacgg 120
   gtgaggaatg gtggggagtt atttttagag cggtgaggaa ggtgggcagg cagcaggtgt 180
   tggcgctcta aaaataactc ccgggagtta tttttagagc ggaggaatgg tggacaccca 240
   aatatggcga cggttcctca cccgtcgcca tatttgggtg tccgccctcg gccggggccg 300
   cattcctggg ggccgggcgg tgctcccgcc cgcctcgata aaaggctccg gggccggcgg 360
   cggcccacga gctacccgga ggagcgggag gcgccaagct ctagaactag tggatcccaa 420
   ggcccaactc cccgaaccac tcagggtcct gtggacagct cacctagctg ccatggtgct 480
   ctgggtgttc ttctttgtga tcctcaccct cagcaacagc tcccactgct ccccacctcc 540
   ccctttgacc ctcaggatgc ggcggtatat cgatgccatc ttcaccaaca gctaccggaa 600
   ggtgctggcc cagctgtccg cccgcaagct gctccaggac atcctgaaca ggcagcaggg 660
   agagaggaac caagagcaag gagcataatg actgcaggaa ttcgatatca agcttatcgg 720
   ggtggcatcc ctgtgacccc tccccagtgc ctctcctggc cctggaagtt gccactccag 780
   tgcccaccag ccttgtccta ataaaattaa gttgcatcat tttgtctgac taggtgtcct 840
   tctataatat tatggggtgg aggggggtgg tatggagcaa ggggcaagtt gggaagacaa 900
   cctgtagggc ctgcggggtc tattgggaac caagctggag tgcagtggca caatcttggc 960
   tcactgcaat ctccgcctcc tgggttcaag cgattctcct gcctcagcct cccgagttgt 1020
   tgggattcca ggcatgcatg accaggctca gctaattttt gtttttttgg tagagacggg 1080
   gtttcaccat attggccagg ctggtctcca actcctaatc tcaggtgatc tacccacctt 1140
   ggcctcccaa attgctggga ttacaggcgt gaaccactgc tcccttccct gtccttctga 1200
   ttttaaaata actataccag caggaggacg tccagacaca gcataggcta cctggccatg 1260
   cccaaccggt gggacatttg agttgcttgc ttggcactgt cctctcatgc gttgggtcca 1320
   ctcagtagat gcctgttgaa ttcgataccg tcgacctcga ggggggggcc ggtaccagct 1380
   tttgttccct ttagtgaggg ttaatttcga gcttggcgta atcatggtca tagctgtttc 1440
   ctgtgtgaaa ttgttatccg ctcacaattc cacacaacat acgagccgga agcataaagt 1500
   gtaaagcctg gggtgcctaa tgagtgagct aactcacatt aattgcgttg cgctcactgc 1560
   ccgctttcca gtcgggaaac ctgtcgtgcc agctgcatta atgaatcggc caacgcgcgg 1620
   ggagaggcgg tttgcgtatt gggcgctctt ccgcttcctc gctcactgac tcgctgcgct 1680
   cggtcgttcg gctgcggcga gcggtatcag ctcactcaaa ggcggtaata cggttatcca 1740
   cagaatcagg ggataacgca ggaaagaaca tgtgagcaaa aggccagcaa aaggccagga 1800
   accgtaaaaa ggccgcgttg ctggcgtttt tccataggct ccgcccccct gacgagcatc 1860
   acaaaaatcg acgctcaagt cagaggtggc gaaacccgac aggactataa agataccagg 1920
   cgtttccccc tggaagctcc ctcgtgcgct ctcctgttcc gaccctgccg cttaccggat 1980
   acctgtccgc ctttctccct tcgggaagcg tggcgctttc tcatagctca cgctgtaggt 2040
   atctcagttc ggtgtaggtc gttcgctcca agctgggctg tgtgcacgaa ccccccgttc 2100
   agcccgaccg ctgcgcctta tccggtaact atcgtcttga gtccaacccg gtaagacacg 2160
   acttatcgcc actggcagca gccactggta acaggattag cagagcgagg tatgtaggcg 2220
   gtgctacaga gttcttgaag tggtggccta actacggcta cactagaaga acagtatttg 2280
   gtatctgcgc tctgctgaag ccagttacct tcggaaaaag agttggtagc tcttgatccg 2340
   gcaaacaaac caccgctggt agcggtggtt tttttgtttg caagcagcag attacgcgca 2400
   gaaaaaaagg atctcaagaa gatcctttga tcttttctac ggggtctgac gctcagaaga 2460
   actcgtcaag aaggcgatag aaggcgatgc gctgcgaatc gggagcggcg ataccgtaaa 2520
   gcacgaggaa gcggtcagcc cattcgccgc caagctcttc agcaatatca cgggtagcca 2580
   acgctatgtc ctgatagcgg tccgccacac ccagccggcc acagtcgatg aatccagaaa 2640
   agcggccatt ttccaccatg atattcggca agcaggcatc gccatgggtc acgacgagat 2700
   cctcgccgtc gggcatgcgc gccttgagcc tggcgaacag ttcggctggc gcgagcccct 2760
   gatgctcttc gtccagatca tcctgatcga caagaccggc ttccatccga gtacgtgctc 2820
   gctcgatgcg atgtttcgct tggtggtcga atgggcaggt agccggatca agcgtatgca 2880
   gccgccgcat tgcatcagcc atgatggata ctttctcggc aggagcaagg tgagatgaca 2940
   ggagatcctg ccccggcact tcgcccaata gcagccagtc ccttcccgct tcagtgacaa 3000
   cgtcgagcac agctgcgcaa ggaacgcccg tcgtggccag ccacgatagc cgcgctgcct 3060
   cgtcctgcag ttcattcagg gcaccggaca ggtcggtctt gacaaaaaga accgggcgcc 3120
   cctgcgctga cagccggaac acggcggcat cagagcagcc gattgtctgt tgtgcccagt 3180
   catagccgaa tagcctctcc acccaagcgg ccggagaacc tgcgtgcaat ccatcttgtt 3240
   caatcatgcg aaacgatcct catcctgtct cttgatcaga tcttgatccc ctgcgccatc 3300
   agatccttgg cggcaagaaa gccatccagt ttactttgca gggcttccca accttaccag 3360
   agggcgcccc agctggcaat tccggttcgc ttgctgtcca taaaaccgcc cagtctagca 3420
   actgttggga agggcgatcg gtgcgggcct cttcgctatt acgccagctg gcgaaagggg 3480
   gatgtgctgc aaggcgatta agttgggtaa cgccagggtt ttcccagtca cgac 3534
<210> 9
   <211> 3534
   <212> DNA
   <213> artificial sequence
<220>
   <223> This is the nucleic acid sequence for the operatively linked comp onents of the 15/27/28 GHRH plasmid.
<400> 9
   gttgtaaaac gacggccagt gaattgtaat acgactcact atagggcgaa ttggagctcc 60
   accgcggtgg cggccgtccg ccctcggcac catcctcacg acacccaaat atggcgacgg 120
   gtgaggaatg gtggggagtt atttttagag cggtgaggaa ggtgggcagg cagcaggtgt 180
   tggcgctcta aaaataactc ccgggagtta tttttagagc ggaggaatgg tggacaccca 240
   aatatggcga cggttcctca cccgtcgcca tatttgggtg tccgccctcg gccggggccg 300
   cattcctggg ggccgggcgg tgctcccgcc cgcctcgata aaaggctccg gggccggcgg 360
   cggcccacga gctacccgga ggagcgggag gcgccaagct ctagaactag tggatcccaa 420
   ggcccaactc cccgaaccac tcagggtcct gtggacagct cacctagctg ccatggtgct 480
   ctgggtgttc ttctttgtga tcctcaccct cagcaacagc tcccactgct ccccacctcc 540
   ccctttgacc ctcaggatgc ggcggtatat cgatgccatc ttcaccaaca gctaccggaa 600
   ggtgctggcc cagctgtccg cccgcaagct gctccaggac atcctgaaca ggcagcaggg 660
   agagaggaac caagagcaag gagcataatg actgcaggaa ttcgatatca agcttatcgg 720
   ggtggcatcc ctgtgacccc tccccagtgc ctctcctggc cctggaagtt gccactccag 780
   tgcccaccag ccttgtccta ataaaattaa gttgcatcat tttgtctgac taggtgtcct 840
   tctataatat tatggggtgg aggggggtgg tatggagcaa ggggcaagtt gggaagacaa 900
   cctgtagggc ctgcggggtc tattgggaac caagctggag tgcagtggca caatcttggc 960
   tcactgcaat ctccgcctcc tgggttcaag cgattctcct gcctcagcct cccgagttgt 1020
   tgggattcca ggcatgcatg accaggctca gctaattttt gtttttttgg tagagacggg 1080
   gtttcaccat attggccagg ctggtctcca actcctaatc tcaggtgatc tacccacctt 1140
   ggcctcccaa attgctggga ttacaggcgt gaaccactgc tcccttccct gtccttctga 1200
   ttttaaaata actataccag caggaggacg tccagacaca gcataggcta cctggccatg 1260
   cccaaccggt gggacatttg agttgcttgc ttggcactgt cctctcatgc gttgggtcca 1320
   ctcagtagat gcctgttgaa ttcgataccg tcgacctcga gggggggccc ggtaccagct 1380
   tttgttccct ttagtgaggg ttaatttcga gcttggcgta atcatggtca tagctgtttc 1440
   ctgtgtgaaa ttgttatccg ctcacaattc cacacaacat acgagccgga agcataaagt 1500
   gtaaagcctg gggtgcctaa tgagtgagct aactcacatt aattgcgttg cgctcactgc 1560
   ccgctttcca gtcgggaaac ctgtcgtgcc agctgcatta atgaatcggc caacgcgcgg 1620
   ggagaggcgg tttgcgtatt gggcgctctt ccgcttcctc gctcactgac tcgctgcgct 1680
   cggtcgttcg gctgcggcga gcggtatcag ctcactcaaa ggcggtaata cggttatcca 1740
   cagaatcagg ggataacgca ggaaagaaca tgtgagcaaa aggccagcaa aaggccagga 1800
   accgtaaaaa ggccgcgttg ctggcgtttt tccataggct ccgcccccct gacgagcatc 1860
   acaaaaatcg acgctcaagt cagaggtggc gaaacccgac aggactataa agataccagg 1920
   cgtttccccc tggaagctcc ctcgtgcgct ctcctgttcc gaccctgccg cttaccggat 1980
   acctgtccgc ctttctccct tcgggaagcg tggcgctttc tcatagctca cgctgtaggt 2040
   atctcagttc ggtgtaggtc gttcgctcca agctgggctg tgtgcacgaa ccccccgttc 2100
   agcccgaccg ctgcgcctta tccggtaact atcgtcttga gtccaacccg gtaagacacg 2160
   acttatcgcc actggcagca gccactggta acaggattag cagagcgagg tatgtaggcg 2220
   gtgctacaga gttcttgaag tggtggccta actacggcta cactagaaga acagtatttg 2280
   gtatctgcgc tctgctgaag ccagttacct tcggaaaaag agttggtagc tcttgatccg 2340
   gcaaacaaac caccgctggt agcggtggtt tttttgtttg caagcagcag attacgcgca 2400
   gaaaaaaagg atctcaagaa gatcctttga tcttttctac ggggtctgac gctcagaaga 2460
   actcgtcaag aaggcgatag aaggcgatgc gctgcgaatc gggagcggcg ataccgtaaa 2520
   gcacgaggaa gcggtcagcc cattcgccgc caagctcttc agcaatatca cgggtagcca 2580
   acgctatgtc ctgatagcgg tccgccacac ccagccggcc acagtcgatg aatccagaaa 2640
   agcggccatt ttccaccatg atattcggca agcaggcatc gccatgggtc acgacgagat   2700
   cctcgccgtc gggcatgcgc gccttgagcc tggcgaacag ttcggctggc gcgagcccct   2760
   gatgctcttc gtccagatca tcctgatcga caagaccggc ttccatccga gtacgtgctc   2820
   gctcgatgcg atgtttcgct tggtggtcga atgggcaggt agccggatca agcgtatgca   2880
   gccgccgcat tgcatcagcc atgatggata ctttctcggc aggagcaagg tgagatgaca   2940
   ggagatcctg ccccggcact tcgcccaata gcagccagtc ccttcccgct tcagtgacaa   3000
   cgtcgagcac agctgcgcaa ggaacgcccg tcgtggccag ccacgatagc cgcgctgcct   3060
   cgtcctgcag ttcattcagg gcaccggaca ggtcggtctt gacaaaaaga accgggcgcc   3120
   cctgcgctga cagccggaac acggcggcat cagagcagcc gattgtctgt tgtgcccagt   3180
   catagccgaa tagcctctcc acccaagcgg ccggagaacc tgcgtgcaat ccatcttgtt   3240
   caatcatgcg aaacgatcct catcctgtct cttgatcaga tcttgatccc ctgcgccatc   3300
   agatccttgg cggcaagaaa gccatccagt ttactttgca gggcttccca accttaccag   3360
   agggcgcccc agctggcaat tccggttcgc ttgctgtcca taaaaccgcc cagtctagca   3420
   actgttggga agggcgatcg gtgcgggcct cttcgctatt acgccagctg gcgaaagggg   3480
   gatgtgctgc aaggcgatta agttgggtaa cgccagggtt ttcccagtca cgac         3534